# EUROPEAN PATENT SPECIFICATION

(11) **EP 2 011 522 B1**
(45) Date of publication and mention of the grant of the patent: **15.12.2010**
(21) Application number: 07111659.4
(22) Date of filing: 03.07.2007
(51) Int. Cl.: A61L 9/12, B05B 17/06

(54) **Wick and wick holder for piezoelectric spray device**
Docht und Dochthalterung für ein piezoelektrisches Sprühgerät
Mèche et support de mèche pour pulvérisateur piezoélectrique

(43) Date of publication of application: 07.01.2009
(73) Proprietor: Microflow Engineering SA, 2034 Peseux (CH)
(72) Inventor: Hess, Joseph, 2022, Bevaix (CH); Feriani, Amir, 2012, Auvernier (CH)
(74) Representative: Balsters, Robert

(56) References cited:
- EP-A- 1 031 446
- EP-A- 1 103 479
- WO-A-2004/056492
- WO-A-2006/114013
- ES-A1- 2 137 108
- US-A1- 2002 136 542
- US-B1- 6 619 560

## Description

The present invention relates to the atomization of liquids in a liquid droplet dispensing device, and more specifically to a wicking apparatus for such a device according to the features of the preamble of claim 1. A wicking apparatus comprising these features is known from EP 1103479. Such droplet dispensing devices are also sometimes called atomizers, nebulizers and the like. They normally contain a nozzle body on a support part, in particular, a nozzle body of a liquid droplet spray device which dispenses a liquid substance as a liquid droplet spray from the device through the nozzles of the nozzle body. They further consist of an actuator based on a vibrating element which generally causes the liquid to vibrate, to be accelerated and expelled as droplets. They further consist of elements such as liquid space, liquid feed and fluid interface to a reservoir, a reservoir as well as electrical connections between the vibrating element and a corresponding electronic circuitry. Such elements may be contained in the aforementioned support part, in a further support part or they may be contained in a number of support parts. The liquid may be for example an ambient fragrance, a perfume, an insecticide, a liquid pharmaceutical formulation, aqueous based liquids and flammable or combustible liquids.

Such nozzle bodies are sometimes called aperture plates, nozzle arrays, dosing apertures, orifice plates, vibratable membrane members, dosing aperture arrangements, aerosol generators and the like. These terms are hence to be understood as being interchangeable throughout the present document.

Such nozzle bodies and droplet spray devices are well known as such. For example, the document WO2007/062698, in the name of the present Applicant, describes a liquid droplet spray device having a top substrate formed of a main body and of a nozzle body. The nozzle body contains a nozzle array of liquid droplet outlet means allowing a liquid substance contained in the liquid droplet spray device to exit the device, in this case as a spray of droplets. Liquid may be supplied from a replaceable reservoir by way of a wick using capillary flow. Generally, the reservoir is a dispensable refill that needs to be replaced regularly.

The document US 7,017,829 describes a liquid droplet spray device having a wicking apparatus with a wick for use in a replaceable reservoir assembly that contains liquid to be atomized by a vibratory aperture plate configured to dispense the liquid from the reservoir assembly through the orifices of the vibratory plate. In this device, the wick must contact the vibratory plate to allow for capillary flow of liquid from the reservoir to the plate for ejection of the liquid.

Thus, in order to ensure capillary flow, typically, the wick is too long, so that any fabrication tolerance, which might lead to a wick that would be too short, will be overcompensated.

Furthermore, in order to obtain an acceptable draining ratio of the reservoir, the wick should fully contact the inner bottom surface of the reservoir for it to be capable to substantially drain and empty the reservoir. Indeed, if the wick does not reach the plate, no spray can be generated or ejected.

To ensure such contact, again, the wick is typically over-sized so as to compensate for manufacturing tolerances.

However, when a wick that is too long than actually designed is inserted into the reservoir, and positioned below the orifice plate, the wick will inevitably be compressed against the plate and against the inner bottom surface of the reservoir. This compression of the wick then leads to unreliable priming and irregular ejection of liquid each time a reservoir is changed.

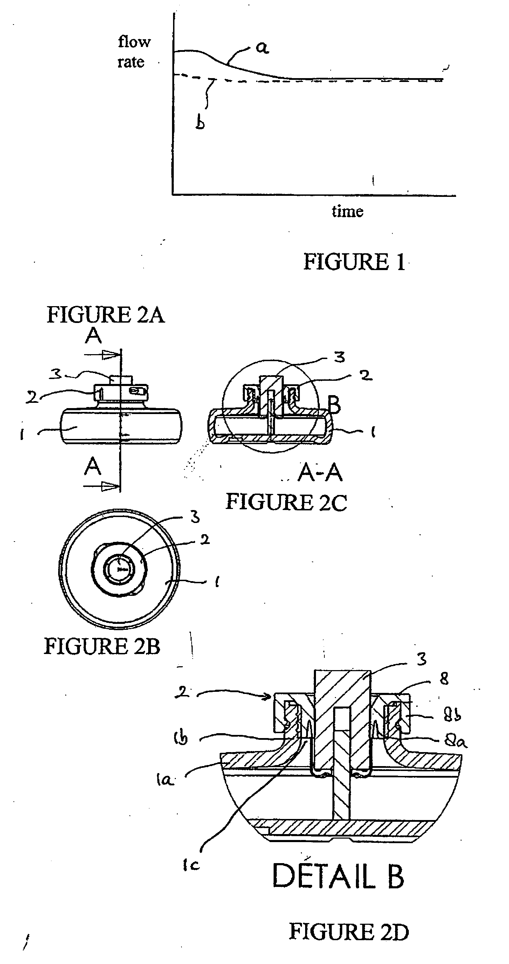

In fact, by compressing the wick, which is thus forced into contact to the plate, a so-called squeezing effect is obtained which leads to an increased flow rate of liquid when the wick is first inserted into a replacement reservoir. However, after a certain moment in time, as shown by curve a in Figure 1, the wick and plate assembly will stabilize in the new position and the flow rate will drop from the increased rate to the intended rate.

Clearly, this is highly undesirable for a consumer, as s/he will have to adjust and re-adjust the flow rate each time the reservoir is replaced in order to have a constant flow rate over time.

Document WO 2005/097349 describes a similar liquid droplet spray device with a wicking apparatus. The wick is formed of two different materials with a rigid main section and a compliant top section. The top compliant section is thus compressible and is pressed into contact with the vibratory orifice plate to allow liquid to flow from the reservoir to the plate by capillary action, and may then be expelled.

Thus, again, the wick is compressed against the plate thus resulting in a squeezing effect, albeit more moderate as compared with the previous document mentioned above, so that here too, an unstable flow rate will occur when inserting a new reservoir.

As for the draining ratio, in this device the rigid main section of the wick would also have to be longer than designed to compensate for manufacturing tolerances and to ensure a full contact with the inner bottom surface of the reservoir.

It is also known to use wick holders so as to correctly and/or securely fix a wick in a reservoir. For example, the document US 6,896,196 describes a liquid droplet spray device with a wicking apparatus that has a wick holder 8 for receiving a wick. The holder is positioned onto a reservoir's bottleneck, and holds the wick in place by way of clamping fingers so as to allow for easy insertion of the wick into the reservoir, while at the same time preventing accidental removal of the wick. However, this document is silent about flow rate or draining ratios.

In view of the above, it is an object of the present invention to provide an innovative wicking apparatus for a droplet spray device that overcomes the inconveniences presented by the prior art documents.

Further, the proposed wicking apparatus can ensure a constant flow rate even after replacing the reservoir as shown by curve b in Figure 1, while at the same time allowing for a high draining ratio despite existing manufacturing tolerances of wicks.

Thus, the present invention concerns a wicking apparatus as defined in the appended claims.

Thanks to the construction of the innovative and inventive wicking apparatus according to the present invention an efficient device fulfilling these objectives may be obtained in a relatively simple and inexpensive manner.

Other features and advantages of the wicking apparatus according to the present invention will become clear from reading the following description, which is given solely by way of a non-limitative example thereby referring to the attached drawings in which:
FIGURE 1, already mentioned, shows a schematic representation of flow rate versus time in view of a squeezing effect by curve a in conventional devices and by curve b in view of the present invention,
FIGURES 2 show an example of the wicking apparatus according to the present invention,
FIGURE 3 shows a detailed top-side view of the wick holder shown in Figure 2,
FIGURE 4 shows a detailed side view of the wick holder shown in Figure 2,
FIGURE 5 shows an example of another reservoir for the wicking apparatus according to the present invention,
FIGURE 6 shows an example of an alternative wick in the wicking apparatus according to the present invention,
FIGURE 7 sows an example of a liquid droplet spray device to which a wicking apparatus according to the preset invention may be mounted, and
FIGURE 8 shows another example of a liquid droplet spray device to which a wicking apparatus according to the preset invention may be mounted.

A preferred embodiment will now be described.

Figure 2 shows an example of the wicking apparatus according to the present invention. The wicking apparatus comprises a reservoir 1, a wick holder 2 and a wick 3. Figure 2a shows a general view of the wicking apparatus, whereas Figure 2b shows a top view thereof. Figure 2c shows a cross-sectional view along lines A-A of Figure 2a, and Figure 2d shows a detailed view of section B of Figure 2c.

As can be seen in these figures, reservoir 1 has, in this example, a general bottle shape, with a main body 1a, and a bottle neck 1b. Wick holder 2 is fitted over the bottleneck 1b, and rests thus thereon. A wick 3 is fitted into the wick holder and enters the reservoir to complete the device according to the present invention. Wick 3 extends beyond the top surface of wick holder so as to enter into capillary contact with a liquid receiving means of a liquid droplet spray device so as to allow for ejection of the liquid as a spray of droplets. As will be understood from the following description, this contact is purely a capillary contact, thus avoiding any compression of wick 3 and thus avoiding the so-called squeezing effect which deteriorates stable functioning of prior art devices when an empty reservoir is replaced by a new full one.

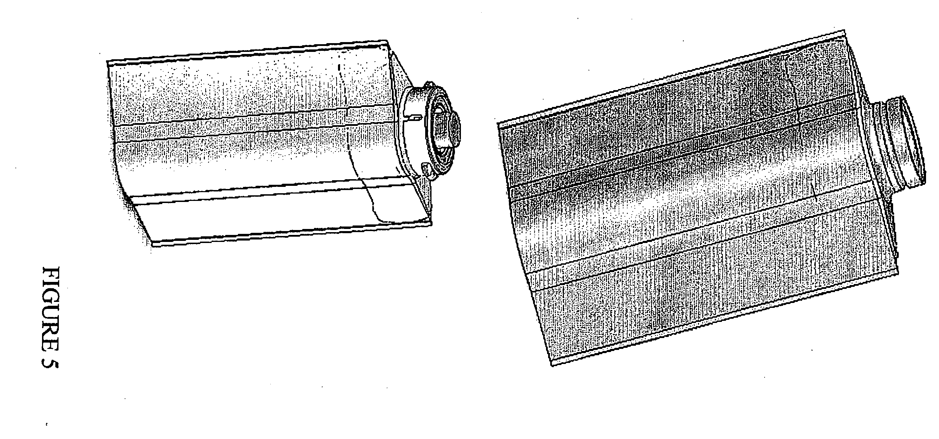

Instead of a bottle shape, reservoir 1 may also be a bag-like reservoir, as shown for example in Figure 5. Such bag-like reservoir can also be a collapsible bag, as is well known in the art.

Thus, in general, reservoir 1 has a reservoir body 1a for containing the volatile substance, and a reservoir neck 1b extending from the reservoir body and terminating in a reservoir opening 1c (see Figure 2D) into which a wick holder and a wick may be inserted.

As shown in detail in Figure 2d, wick holder 2 has a top part 8 for fitting over reservoir neck 1b, similar to a bottle cap, and a bottom part 10 for insertion into the reservoir opening. Top part 8 is generally flat and plate-formed with a central opening 6 for receiving a wick. At the peripheral edge of top part 8, two projections 8a, 8b are provided which are spaced apart and which extend downward from the plate towards reservoir body la when wick holder 2 is fitted onto reservoir 1. In this example, these projections follow substantially the entire periphery of top part 8.

First projection 8a is arranged on an inner periphery with respect to the edge of top part 8, and is arranged to contact an inner wall of reservoir neck 1b. Second projection 8b substantially follows the edge of top part 8 in this example, and is arranged to contact an outer wall of reservoir neck 1b. In this way, top part 8 sits on the reservoir supported by reservoir neck 1b and held in place by first and second projections 8a, 8b which pinch together reservoir neck 1b.

This arrangement allows for a precise fitting of wick holder 2 on reservoir 1, as the top of reservoir neck constitutes a reference surface.

By using a material for wick holder 2 that can be manufactured with high precision, for example by using a plastic that can be injection-moulded, distances with respect to this reference point can be clearly and accurately defined.

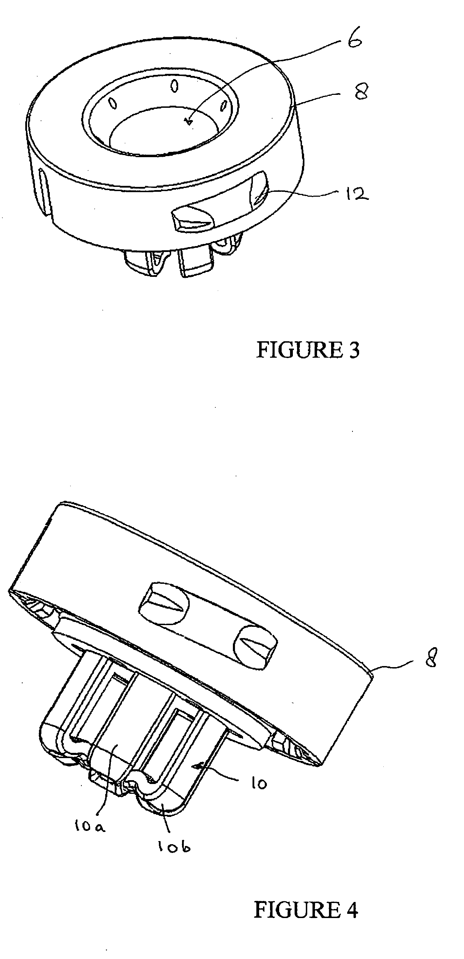

As shown in Figure 3, elastic detachment means 12 may be provided on a side surface of second projection 8b to allow for secure attachment to reservoir neck 1b and for easy removal there from. Such means are well known as such in the art and will not be discussed in more detail here.

Figure 4 shows a detailed side view of the wick holder shown in Figure 2. As can be seen in this figure, first projection 8a interfaces with integrally formed bottom part 10 that continues further downwards by way of a plurality of wick supporting arms 10a spaced apart along the inner edge periphery of first projection 8a. These supporting arms 10a extend further downwards away from top part 8 and each have a flexible end portion 10b projecting inwardly towards the centre of the reservoir opening. These flexible end portions are arranged to allow for a slight vertical displacement by deformation, as will be explained in more detail further on.

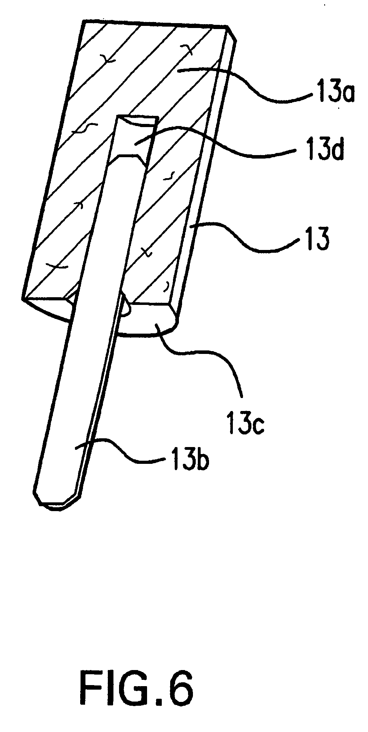

As shown in Figure 2d and Figure 6, wick 3 is step-shaped having a larger diameter upper part 3a for supplying by capillary contact liquid to the liquid receiving section of a liquid droplet spray device when mounted thereto, and a smaller diameter lower part 3b, as compared to the larger diameter upper part 3a, for extending into the reservoir 1 to contact the inner bottom surface thereof when inserted. As can be seen in Figure 2d, the interface between larger diameter upper part 3a and smaller diameter lower part 3b defines a shoulder 3c. When wick 3 is inserted into wick holder 2, his shoulder is supported and held in place by the flexible end portions 10b of one or more supporting arms 10a of wick holder 8.

Flexible end portions 10b can slightly move downwards when a certain weight or pressure is applied thereto. This displacement is larger than the typical fabrication tolerance of a wick. Generally, a wick can be fabricated with a tolerance of its length of around ± 0.4 mm. The displacement of the flexible end portion is then chosen, for example, to be 1 mm to allow to fully absorb the manufacturing tolerances. Thus, for example, if a wick is supposed to be 12.7 mm long, in reality it may be between 12.3 and 13.1 mm. A conventional wicking apparatus would then have a wick manufactured with a specified length of at least 13.1 mm, so as to be certain that the wick will be long enough to reach both the bottom inner surface of the reservoir, and to touch the liquid receiving section for inputting the liquid into the spray device.

Thus, typically such a conventional wicking apparatus will use a wick that is too long, and will thus compress the wick into place.

However, according to the present invention, a wick is specified for manufacturing for the correct designed length of 12.7 mm. Such wick will not be compressed, but instead, the flexible end portions 10b will move downwards to compensate for any excess length due to manufacturing tolerances.

In fact, the flexible end portions are arranged at a distance such that even a wick with a minimum length of, in this example, 12.7 - 0.4 = 12.3 mm will arrive at the correct level above wick holder 2 so as to ensure a capillary contact with the liquid receiving section of a spray device. Any longer wick will cause the flexible end portions to lower so as to absorb the excess length, so that the wick will always arrive at the correct level above wick holder 2 so as to ensure a purely capillary contact with the liquid receiving section of a spray device, free from compression, and thus free from any squeezing effect. If, due to tolerances, the wick is too short, the flexible end portion 10b will also compensate such that the wick will always arrive at the correct level above the wick holder.

Figure 6 shows an example of an alternative wick in the wicking apparatus according to the present invention. In this alternative arrangement, wick 13 also is step-shaped having a larger diameter upper part 13a for supplying by capillary contact liquid to the liquid receiving section of a liquid droplet spray device when mounted thereto, and a smaller diameter lower part 13b, as compared to the larger diameter upper part 3a, for extending into the reservoir 1 to contact the inner bottom surface thereof when inserted. Again, the interface between larger diameter upper part 13a and smaller diameter lower part 13b defines a shoulder 13c. However, these two parts 13a, 13b are two separate parts, instead of being formed integrally as described above. Larger diameter upper part 13a has a central hole 13d for receiving smaller diameter lower part 13b therein in a slideable manner. S such, the length of wick 13 may vary, and any fabrication tolerances can also be absorbed by the inner relative displacement of smaller diameter lower part 13b with respect larger diameter upper part 13a.

By combining such a wick 13, with the wick holder 2 described above, any manufacturing tolerance can be compensated for, by the flexible end portions, by the relative displacement of the wick portions, or by a combination of these two.

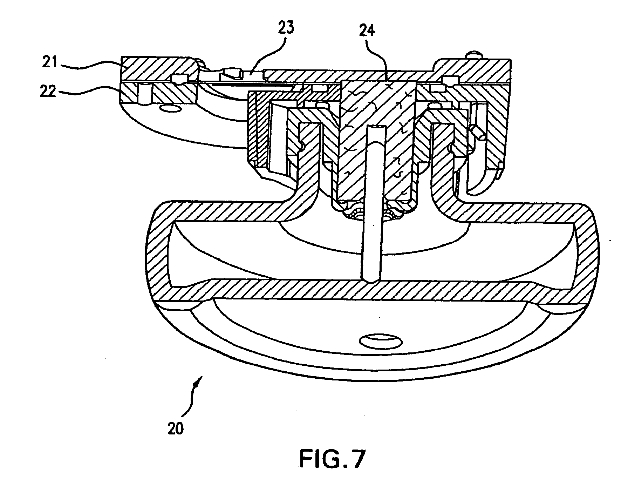

Figure 7 sows an example of a liquid droplet spray device to which a wicking apparatus according to the preset invention may be mounted. As can be seen, liquid droplet spray device 20 comprises a top package 21, a bottom package 22, liquid outlet means 23, and liquid inlet means 24. More details of this device are described in above-mentioned document WO2007/062698 in the name of the present Applicant Actuating means, such as a piezoelectric vibrating means, not shown, are provided to actuate any liquid present in the liquid outlet means. Liquid outlet means comprises a nozzle array for allowing liquid to exit the spray device once excited. By mounting the wicking apparatus according to the present invention, wick 3, 13 will contact the liquid receiving section of liquid inlet means 24 to allow liquid to enter the spray device by capillary action only. Once the liquid enters the spray device, it is transported to the liquid outlet means 23. Here, the liquid undergoes a vibration, by way of the actuating means, not shown, to excite the liquid so as to expel it through outlet nozzles provide din the outlet means to generate a spray of droplets.

Thanks to the present invention, the wick will always be correctly positioned in the liquid receiving means, and will have a capillary contact therewith that is free from compression, despite any manufacturing tolerances of the wick length. This ensures a stable flow rate (curve b in Figure 1).

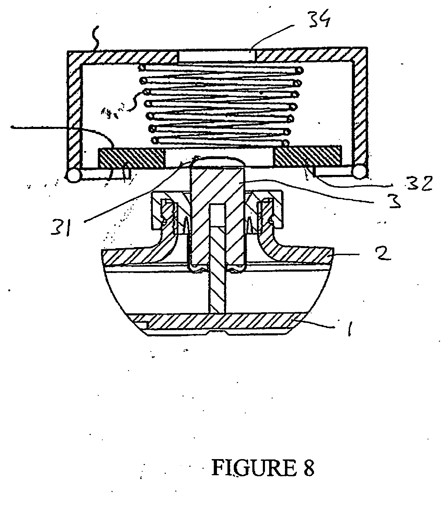

Figure 8 shows another example of a liquid droplet spray device to which a wicking apparatus according to the present invention may be mounted. In this spray device, the liquid outlet means take the form of a vibratory dome-shaped nozzle plate, which may be similar to above-mentioned document US 7,017,829. Thus, the liquid inlet means 31 are arranged centrically below the liquid outlet means 34 in that the wick transports the liquid to the bottom of the vibratory plate, which is then excited by suitable vibrating means, for example, circular vibrating element 32 to suck in the liquid into the nozzles of the nozzle plate 31 and to expel the liquid as a spray of droplets through liquid outlet means 34.

Having described now the preferred embodiment of this invention, it will be apparent to one of skill in the art that other embodiments incorporating its concept may be used. It is felt, therefore, that this invention should not be limited to the disclosed embodiment, but rather should be limited only by the scope of the appended claims.

## Claims

1. Wicking apparatus for volatile substances that may be ejected as a spray of droplets by a liquid droplet dispenser device when fitted with the wicking apparatus, the wicking apparatus comprising:
a volatile substance reservoir (1),
a wick holder (2), and
a wick (3),
said reservoir (1) having a reservoir body (1a) for containing the volatile substance, and a reservoir neck (1b) extending from the reservoir body and terminating in a reservoir opening (1c) into which a wick holder and a wick may be inserted,
said wick holder (2) having a top part (8) for fitting on said reservoir neck (1b) and a bottom part (10) for insertion into said reservoir opening (1c),
said top part (8) being plate-formed with a central opening (6) for receiving a wick, and with two peripheral projections (8a, 8b) that are spaced apart and that extend downward from the plate towards the reservoir body (1a) when fitted on said reservoir (1) along substantially the entire periphery of said top part (8),
a first projection (8a) of said two projections for contacting an inner wall of said reservoir neck (1b), and
a second projection (8b) of said two projections for contacting an outer wall of said reservoir neck (1b),
so that the top part sits on the reservoir supported by the reservoir neck (1b) and held in place by said first and second projections (8a, 8b),
said bottom part (10) being integrally formed with said first projection (8a) and having a plurality of wick supporting arms (10a) spaced apart along its periphery, said supporting arms (10a) extending further downwards away from said top part (8) and having flexible end portions (10b) projecting inwardly towards the centre of said reservoir opening (1c), **characterised by**
said wick (3) being step-shaped having a larger diameter part (3a) for supplying by capillary contact liquid to a liquid receiving section of said liquid droplet spray device when mounted thereto, and a smaller diameter part (3b), as compared to said larger diameter part (3a), for extending into the reservoir (1) to contact the inner bottom surface thereof when inserted,
the interface between said larger diameter part (3a) and said smaller diameter part (3b) defining a shoulder (3c) which is supported and held in place by the flexible end portions (10b) of said plurality of supporting arms (10a).

2. Wicking apparatus according to claim 1, wherein said reservoir (1) is a bottle.

3. Wicking apparatus according to claim 1, wherein said reservoir (1) is a collapsible bag.

4. Wicking apparatus according to anyone of claims 1 to 3, wherein said wick consists of two separate parts, said larger diameter part having a central opening, and said smaller diameter part being slideably fitted into said larger diameter part.

5. Liquid droplet spray device comprising a nozzle body, a piezo-electric actuator, a fluid chamber located below said nozzle body and a liquid receiving section, and further comprising a wicking apparatus according to anyone of the preceding claims, wherein said liquid receiving section is arranged to receive liquid through capillary contact from said wick, and to provide said chamber with said liquid for ejecting said received liquid as a spray of droplets.

6. Liquid droplet spray device according to claim 5, wherein said liquid receiving section is arranged eccentric with respect to said nozzle body.

7. Liquid droplet spray device according to claim 5, wherein said liquid receiving section is arranged centric with respect to said nozzle body.

8. Liquid droplet spray device according to claim 7, wherein said nozzle body is a vibratory plate.

## Patentansprüche

1. Dochtvorrichtung für flüchtige Substanzen, die von einem Flüssigkeitströpfchen-Verteiler als ein Sprühnebel von Tröpfchen ausgestoßen werden können, wenn der Flüssigkeitströpfchen-Verteiler mit der Dochtvorrichtung versehen ist, mit:
einem Vorratsbehälter (1) für flüchtige Substanzen;
einem Dochthalter (2) und
einem Docht (3),
wobei
der Vorratsbehälter (1) einen Vorratsbehälter-Hauptteil (1 a) zum Aufnehmen der flüchtigen Substanz und einen Vorratsbehälterhals (1 b) hat, der von dem Vorratsbehälter-Hauptteil ausgeht und in einer Vorratsbehälter-Öffnung (1 c) endet, in die der Dochthalter und der Docht eingesteckt werden können,
der Dochthalter (2) einen oberen Teil (8) zum Befestigen an dem Vorratsbehälterhals (1 b) und einen unteren Teil (10) zum Einführen in die Vorratsbehälter-Öffnung (1 c) hat,
der obere Teil (8) die Form einem Scheibe mit einer mittigen Öffnung (6) zum Aufnehmen des Dochtes und mit zwei peripheren vorspringenden Teilen (8a, 8b) hat, die in einem Abstand voneinander angeordnet sind und beim Befestigen an dem Vorratsbehälter (1) entlang im Wesentlichen der gesamten Peripherie des oberen Teils (8) von der Scheibe nach unten zu dem Vorratsbehälter-Hauptteil (1 a) verlaufen, ein erster vorspringender Teil (8a) der beiden vorspringenden Teile zum kontaktieren einer Innenwand des Vorratsbehälterhalses (1 b) und ein zweiter vorspringender Teil (8b) der beiden vorspringenden Teile zum kontaktieren einer Außenwand des Vorratsbehälterhalses (1 b), sodass der obere Teil so auf dem Vorratsbehälter sitzt, dass er von dem Vorratsbehälterhals (1 b) abgestützt wird und von dem ersten und dem zweiten vorspringenden Teil (8a, 8b) an der richtigen Position gehalten wird, und
der untere Teil (10) eine Einheit mit dem ersten vorspringenden Teil (8a) bildet und eine Vielzahl von Docht-Haltearmen (10a) hat, die entlang seiner Peripherie mit Abstand voneinander angeordnet sind, wobei die Haltearme (10a) weiter nach unten weg von dem oberen Teil (8) verlaufen und flexible Endteile (10b) haben, die nach innen zu dem Mittelpunkt der Vorratsbehälter-Öffnung (1 c) vorstehen,
**dadurch gekennzeichnet, dass**
der Docht (3) die Form einer Stufe hat, die einen Teil (3a) mit einem größeren Durchmesser zum Zuführen, durch kapillaren Kontakt, Flüssigkeit zu einem Flüssigkeitsaufnahmeteil des Flüssigkeitströpfchen-Verteilers, wenn er daran angebracht ist, und einen Teil (3b) mit einem kleineren Durchmesser als der Teil (3a) mit dem größeren Durchmesser hat, um beim Einführen so in den Vorratsbehälter (1) hineinzureichen, dass er dessen innere Unterseite berührt, und
die Grenzfläche zwischen dem Teil (3a) mit einem größeren Durchmesser und dem Teil (3b) mit dem kleineren Durchmesser eine Schulter (3c) definiert, die von den flexiblen Endteilen (10b) der Vielzahl von Haltearmen (10a) abgestützt wird und an der richtigen Position gehalten wird.

2. Dochtvorrichtung nach Anspruch 1, **dadurch gekennzeichnet, dass** der Vorratsbehälter (1) eine Flasche ist.

3. Dochtvorrichtung nach Anspruch 1, **dadurch gekennzeichnet, dass** der Vorratsbehälter (1) ein zusammenlegbarer Beutel ist.

4. Dochtvorrichtung nach einem der Ansprüche 1 bis 3, **dadurch gekennzeichnet, dass** der Docht aus zwei getrennten Teilen besteht, wobei der Teil mit dem größeren Durchmesser eine mittige Öffnung hat und der Teil mit dem kleineren Durchmesser gleitfähig in den Teil mit dem größeren Durchmesser eingepasst ist.

5. Flüssigkeitströpfchen-Sprühvorrichtung, die einen Düsenkörper, einen piezoelektrischen Aktuator, eine Fluidkammer, die unter dem Düsenkörper angeordnet ist, und einen Flüssigkeitsaufnahmeteil aufweist und weiterhin eine Dochtvorrichtung nach einem der vorhergehenden Ansprüche aufweist, wobei der Flüssigkeitsaufnahmeteil so eingerichtet ist, dass er eine Flüssigkeit durch Kapillarkontakt von dem Docht aufnimmt und die Kammer mit der Flüssigkeit versorgt, um die aufgenommene Flüssigkeit als einen Sprühnebel von Tröpfchen auszustoßen.

6. Flüssigkeitströpfchen-Sprühvorrichtung nach Anspruch 5, **dadurch gekennzeichnet, dass** der Flüssigkeitsaufnahmeteil in Bezug auf den Düsenkörper außermittig angeordnet ist.

7. Flüssigkeitströpfchen-Sprühvorrichtung nach Anspruch 5, **dadurch gekennzeichnet, dass** der Flüssigkeitsaufnahmeteil in Bezug auf den Düsenkörper mittig angeordnet ist.

8. Flüssigkeitströpfchen-Sprühvorrichtung nach Anspruch 7, **dadurch gekennzeichnet, dass** der Düsenkörper eine Schwingscheibe ist.

## Revendications

1. Appareil à effet mèche pour des substances volatiles qui peuvent être éjectées sous la forme d'une pulvérisation de gouttelettes par un dispositif distributeur de gouttelettes de liquide lorsqu'il est équipé de l'appareil à effet mèche, l'appareil à effet mèche comprenant :
un réservoir (1) de substance volatile,
un porte-mèche (2), et
une mèche (3),
ledit réservoir (1) ayant un corps de réservoir (1a) pour contenir la substance volatile, et un col de réservoir (1b) s'étendant à partir du corps de réservoir et se terminant dans une ouverture de réservoir (1c) dans laquelle un porte-mèche et une mèche peuvent être insérés,
ledit porte-mèche (2) ayant une partie supérieure (8) pour ajustement sur ledit col de réservoir (1b) et une partie inférieure (10) pour insertion dans ladite ouverture de réservoir (1c),
ladite partie supérieure (8) étant en forme de plaque avec une ouverture centrale (6) pour recevoir une mèche, et avec deux saillies périphériques (8a, 8b) qui sont espacées et qui s'étendent vers le bas à partir de la plaque vers le corps de réservoir (1a) lorsqu'elle est ajustée sur ledit réservoir (1) sur substantiellement la totalité de la périphérie de ladite partie supérieure (8),
une première saillie (8a) desdites deux saillies pour être en contact avec une paroi intérieure dudit col de réservoir (1b), et
une deuxième saillie (8b) desdites deux saillies pour être en contact avec une paroi extérieure dudit col de réservoir (1b),
de sorte que la partie supérieure repose sur le réservoir supportée par le col de réservoir (1b) et maintenue en place par lesdites première et deuxième saillies (8a, 8b),
ladite partie inférieure (10) étant formée de façon intégrale avec ladite première saillie (8a) et ayant une pluralité de bras de support de mèche (10a) espacés le long de sa périphérie, lesdits bras de support (10a) s'étendant plus vers le bas à l'opposé de ladite partie supérieure (8) et ayant des parties d'extrémité flexibles (10b) se projetant vers l'intérieur vers le centre de ladite ouverture de réservoir (1c),
**caractérisé par**
ladite mèche (3) étant en forme de marche ayant une partie de diamètre plus grand (3a) pour amener par contact capillaire un liquide jusqu'à une section de réception de liquide dudit dispositif de pulvérisation de gouttelettes de liquide lorsqu'elle est montée sur celui-ci, et une partie de diamètre plus petit (3b), par rapport à ladite partie de diamètre plus grand (3a), pour s'étendre à l'intérieur du réservoir (1) pour être en contact avec la surface intérieure de fond de celui-ci lorsqu'elle est insérée,
l'interface entre ladite partie de diamètre plus grand (3a) et ladite partie de diamètre plus petit (3b) définissant un épaulement (3c) qui est supporté et maintenu en place par les parties d'extrémité flexibles (10b) de ladite pluralité de bras de support (10a).

2. Appareil à effet mèche selon la revendication 1, dans lequel ledit réservoir (1) est une bouteille.

3. Appareil à effet mèche selon la revendication 1, dans lequel ledit réservoir (1) est un sac compressible.

4. Appareil à effet mèche selon l'une quelconque des revendications 1 à 3, dans lequel ladite mèche consiste en deux parties séparées, ladite partie de diamètre plus grand ayant une ouverture centrale, et ladite partie de diamètre plus petit étant ajustée de façon coulissante dans ladite partie de diamètre plus grand.

5. Dispositif de pulvérisation de gouttelettes de liquide comprenant un corps de buse, un actionneur piézoélectrique, une chambre de fluide située en-dessous dudit corps de buse et une section de réception de liquide, et comprenant en outre un appareil à effet mèche selon l'une quelconque des revendications précédentes, dans lequel ladite section de réception de liquide est agencée de façon à recevoir un liquide par contact capillaire de ladite mèche et pour amener jusqu'à ladite chambre ledit liquide pour une éjection dudit liquide reçu sous la forme d'une pulvérisation de gouttelettes.

6. Dispositif de pulvérisation de gouttelettes de liquide selon la revendication 5, dans lequel ladite section de réception de liquide est agencée de façon excentrique par rapport audit corps de buse.

7. Dispositif de pulvérisation de gouttelettes de liquide selon la revendication 5, dans lequel ladite section de réception de liquide est agencée de façon centrique par rapport audit corps de buse.

8. Dispositif de pulvérisation de gouttelettes de liquide selon la revendication 7, dans lequel ledit corps de buse est une plaque vibrante.
